# DEMANDE DE BREVET EUROPEEN

(11) **EP 0 764 723 A1**
(43) Date de publication de la demande: **26.03.1997**
(21) Numéro de dépôt: 96401789.1
(22) Date de dépôt: 14.08.1996
(51) Int. Cl.: C12N 15/74, C12N 1/20, C12N 1/21

(54) **Séquence d'acides nucléiques et plasmides comprenant au moins un mécanisme de résistance aux phages, bactéries les contenant et leur utilisation**

(30) Priorité: 18.08.1995 FR 9509913
(71) Demandeur: SYSTEMS BIO-INDUSTRIES, F-92100 Boulogne (FR)
(72) Inventeur: Prevots, Fabien, 31400 Toulouse (FR); Tolou, Sandrine, 31650 St Orens de Gameville (FR); Daloyau, Marlène, 31320 Castanet (FR)
(74) Mandataire: Gillard, Marie-Louise

(57) **Abrégé**

L'invention a pour objet une séquence d'ADN de 817 pb comprenant au moins un mécanisme de résistance aux phages obtenue à partir de l'ADN total contenue dans la souche Lactococcus lactis ssp cremoris déposée à la C.N.C.M. sous le N° I-943.

LISTE DE SEQUENCES

(1) INFORMATION GENERALE:

(i) DEPOSANT:
(A) NOM: SYSTEMS BIO-INDUSTRIES
(B) RUE: 4, Place des Ailes
(C) VILLE: Boulogne Billancourt
(E) PAYS: France
(F) CODE POSTAL: 92641 Cedex
(G) TELEPHONE: 47 12 25 25
(H) TELECOPIE: 47 12 26 56

(ii) TITRE DE L' INVENTION: Séquence d'acides nucléiques et
plasmides comprenant au moins un mécanisme de résistance
aux phages, bactéries les contenant et leur utilisation

(iii) NOMBRE DE SEQUENCES: 4

(iv) FORME DECHIFFRABLE PAR ORDINATEUR:
(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(vi) DONNEES DE LA DEMANDE ANTERIEURE:
(A) NUMERO DE LA DEMANDE: FR 95 09913
(B) DATE DE DEPOT: 18-AUG-1995

(2) INFORMATION POUR LA SEQ ID NO: 1:

(i) CARACTERISTIQUES DE LA SEQUENCE:
(A) LONGUEUR: 817 paires de bases
(B) TYPE: acide nucléique
(C) NOMBRE DE BRINS: simple
(D) CONFIGURATION: linéaire

(ii) TYPE DE MOLECULE: ADN (génomique)

(iii) HYPOTHETIQUE: NON

(iii) ANTI-SENS: NON

(vi) ORIGINE:
(A) ORGANISME: Lactococcus lactis

(ix) CARACTERISTIQUE ADDITIONELLE:
(A) NOM/CLE: CDS
(B) EMPLACEMENT: 157..690
(xi) DESCRIPTION DE LA SEQUENCE: SEQ ID NO: 1:

GTAAAAAGTA AAAACGTTAG AAATGGTGAT TTATTTTTCT TTTAATTAAT GATATTATTA 60
GTTAATAAAA TTAATTAGGA GATTTAAGTT GTGAAGGATG TTTTGGATTA TATTATTTCT 120
GGTATAAGTA TATGTATATT TATTTTGGCA GTTTAT ATG ATA AAG AAA ATT CCA 174
Met Ile Lys Lys Ile Pro
1               5

GAA ATG GTG AGT GAT AAA TTA AAA AGT GAC AGA GAA TTT GAA TTT AAT 222
Glu Met Val Ser Asp Lys Leu Lys Ser Asp Arg Glu Phe Glu Phe Asn
            10                  15                  20

AAG GAG TTA CAG ATT GAT GAA TTT TAT CGA AAA GAT GGG AAT CTG CAA 270
Lys Glu Leu Gln Ile Asp Glu Phe Tyr Arg Lys Asp Gly Asn Leu Gln
        25                  30                  35

CAG ATT ATG ATG AAC TGG ACC GAA CTT GCA ATT GAT ACA AAT GCA ATG 318
Gln Ile Met Met Asn Trp Thr Glu Leu Ala Ile Asp Thr Asn Ala Met
    40                  45                  50

GAG TCG CTT GAT TCT AAG AAC GGA CAG AAA AAA TTA CGG AAG CTT GTT 366
Glu Ser Leu Asp Ser Lys Asn Gly Gln Lys Lys Leu Arg Lys Leu Val
55                  60                  65                  70

CAA GAA ACA CTT GGA TAT GGT TCA GGA AGA ACA GTT AAA TTA CTA ACA 414
Gln Glu Thr Leu Gly Tyr Gly Ser Gly Arg Thr Val Lys Leu Leu Thr
                75                  80                  85

GAA ATG CTT CAA GAA AGT TAT CGA AGT AAT GAT ACT GAA TCA GAA AAT 462
Glu Met Leu Gln Glu Ser Tyr Arg Ser Asn Asp Thr Glu Ser Glu Asn
            90                  95                  100

ACT GAA TCA GGA AAT AAT GAA TCA GAA AAT AAT GAA TCT ATA AAT AGG 510
Thr Glu Ser Gly Asn Asn Glu Ser Glu Asn Asn Glu Ser Ile Asn Arg
        105                 110                 115

TCT TCT GCC ACT ATA ATG TTG CTG TTG GCA ATG GTT GTT TCT TCT CTA 558
Ser Ser Ala Thr Ile Met Leu Leu Leu Ala Met Val Val Ser Ser Leu
    120                 125                 130

AAG GAA GAT TTT ACT GGA CAA AAA GTT GAC CCA TTA GAT GTC CTT AAA 606
Lys Glu Asp Phe Thr Gly Gln Lys Val Asp Pro Leu Asp Val Leu Lys
135                 140                 145                 150

ATA AAA CTC ACT GAC TAT TAT AAT CAT GAG GGA TTA TTT AAA GAA CTT 654
Ile Lys Leu Thr Asp Tyr Tyr Asn His Glu Gly Leu Phe Lys Glu Leu
                155                 160                 165

TTT GAA AGT GTA AAT AAC AAA CTA GGA GTT GAA GTT TAATAATGGA 700
Phe Glu Ser Val Asn Asn Lys Leu Gly Val Glu Val
            170                 175

AATTAATTTT ATAGCTCTGG CCTTTGCATC TGTTATTGTA GGTGGGGTAT TCATTGGAAT 760
ATTTATTCTG GTTTACAAAT GGTTGAAGAA ATGATAAGTA TAGCTAGACT ATGAAAA 817

(2) INFORMATION POUR LA SEQ ID NO: 2:

(i) CARACTERISTIQUES DE LA SEQUENCE:
(A) LONGUEUR: 178 acides aminés
(B) TYPE: acide aminé
(D) CONFIGURATION: linéaire

(ii) TYPE DE MOLECULE: protéine

(xi) DESCRIPTION DE LA SEQUENCE: SEQ ID NO: 2:

Met Ile Lys Lys Ile Pro Glu Met Val Ser Asp Lys Leu Lys Ser Asp
1               5                   10                  15
Arg Glu Phe Glu Phe Asn Lys Glu Leu Gln Ile Asp Glu Phe Tyr Arg
            20                  25                  30
Lys Asp Gly Asn Leu Gln Gln Ile Met Met Asn Trp Thr Glu Leu Ala
        35                  40                  45
Ile Asp Thr Asn Ala Met Glu Ser Leu Asp Ser Lys Asn Gly Gln Lys
    50                  55                  60
Lys Leu Arg Lys Leu Val Gln Glu Thr Leu Gly Tyr Gly Ser Gly Arg
65                  70                  75                  80
Thr Val Lys Leu Leu Thr Glu Met Leu Gln Glu Ser Tyr Arg Ser Asn
                85                  90                  95

Asp Thr Glu Ser Glu Asn Thr Glu Ser Gly Asn Asn Glu Ser Glu Asn
            100                 105                 110
Asn Glu Ser Ile Asn Arg Ser Ser Ala Thr Ile Met Leu Leu Leu Ala
        115                 120                 125
Met Val Val Ser Ser Leu Lys Glu Asp Phe Thr Gly Gln Lys Val Asp
    130                 135                 140
Pro Leu Asp Val Leu Lys Ile Lys Leu Thr Asp Tyr Tyr Asn His Glu
145                 150                 155                 160
Gly Leu Phe Lys Glu Leu Phe Glu Ser Val Asn Asn Lys Leu Gly Val
                165                 170                 175
Glu Val

(2) INFORMATION POUR LA SEQ ID NO: 3:

(i) CARACTERISTIQUES DE LA SEQUENCE:
(A) LONGUEUR: 31 paires de bases
(B) TYPE: acide nucléique
(C) NOMBRE DE BRINS: simple
(D) CONFIGURATION: linéaire

(ii) TYPE DE MOLECULE: ADN (génomique)

(iii) HYPOTHETIQUE: NON

(ix) CARACTERISTIQUE ADDITIONELLE:
(A) NOM/CLE: misc_signal
(B) EMPLACEMENT: 6..11
(D) AUTRES RENSEIGNEMENTS: /function= "site de restriction
EcoRI"

(ix) CARACTERISTIQUE ADDITIONELLE:
(A) NOM/CLE: misc_structure
(B) EMPLACEMENT: 12..31
(D) AUTRES RENSEIGNEMENTS: /function= "séquence homologue
aux nucléotides 1-20 de SED ID NO:1"

(xi) DESCRIPTION DE LA SEQUENCE: SEQ ID NO: 3:

TACGTGAATT CGTAAAAAGT AAAAACGTTA G                                31

(2) INFORMATION POUR LA SEQ ID NO: 4:

(i) CARACTERISTIQUES DE LA SEQUENCE:

(A) LONGUEUR: 31 paires de bases
(B) TYPE: acide nucléique
(C) NOMBRE DE BRINS: simple
(D) CONFIGURATION: linéaire

(ii) TYPE DE MOLECULE: ADN (génomique)

(iii) HYPOTHETIQUE: NON

(ix) CARACTERISTIQUE ADDITIONELLE:
(A) NOM/CLE: misc_signal
(B) EMPLACEMENT: 6..11
(D) AUTRES RENSEIGNEMENTS: /function= "site de restriction
EcoRI"

(ix) CARACTERISTIQUE ADDITIONELLE:
(A) NOM/CLE: misc_structure
(B) EMPLACEMENT: 12..31
(D) AUTRES RENSEIGNEMENTS: /function= "séquence homolgue à
l'ADNc correspondant aux nucléotides 798-817
de SEQ ID NO:1"

(xi) DESCRIPTION DE LA SEQUENCE: SEQ ID NO: 4:

TACGTGAATT CTTTTCATAG TCTAGCTATA C 31

## Description

La présente invention a pour objet une nouvelle séquence d'acides nucléiques et des plasmides susceptibles de s'hybrider avec celle-ci, porteurs d'au moins un mécanisme de résistance aux phages, les bactéries lactiques contenant cette séquence ou ces plasmides, en particulier les lactocoques appartenant à l'espèce Lactococcus lactis, l'utilisation de certaines souches de ces lactocoques pour transférer, notamment par conjugaison, un mécanisme de résistance aux phages à des souches d'intérêt industriel, en particulier dans l'industrie laitière, et à l'utilisation de certaines souches de Lactococcus lactis pour obtenir ces plasmides.

Les bactéries lactiques sont impliquées dans l'élaboration et la conservation d'un grand nombre de produits alimentaires, tels que les fromages, le beurre, les yaourts, le saucisson ou la choucroute. Parmi ceux-ci les produits laitiers occupent une place particulièrement importante. La transformation industrielle du lait est faite dans des cuves de fermentation de plus en plus grandes, dans lesquelles l'apparition de phages des bactéries lactiques peut avoir des conséquences graves, voire catastrophiques : variation des caractéristiques, notamment organoleptiques, du produit final ; perte du produit présent dans la cuve et nécessité de décontaminer cette dernière ainsi que les installations environnantes. Il existe donc dans l'industrie laitière un besoin impérieux de nouveaux moyens et de nouvelles méthodes permettant de rendre les bactéries lactiques plus résistantes aux phages.

Les phages des bactéries lactiques appartiennent à trois grands groupes d'homologie (I), (II) et (III) définis par des études d'hybridation ADN/ADN selon RELANO P. et al (1987), J. Gen. Microbiol. 133, 3053-3063. Les groupes (I) et (III) comprennent uniquement des phages virulents. Le groupe (II) comprend des phages virulents et des phages tempérés. A l'intérieur d'un même groupe les homologies sont fortes et, d'un groupe à l'autre, les homologies sont très faibles. Les phages du groupe (I) ont une nucléocapside oblongue alors que les phages des groupes (II) et (III) ont une nucléocapside isométrique.

On sait qu'il existe plusieurs mécanismes naturels de résistance aux phages dont les trois principaux sont :
- l'inhibition de l'adsorption des phages ; dans ce mécanisme, l'adsorption du phage par la bactérie est inhibée ou retardée.
- le système restriction/modification ; ce système fait intervenir une enzyme de restriction qui dégrade l'ADN du phage dès son entrée dans la bactérie.
- l'infection abortive ; selon ce troisième mécanisme, l'adsorption des phages est normale, mais leur multiplication ne se produit pas.

Ces mécanismes sont décrits en détail par SANDERS M. dans Biochimie 70, (1988), 411-421.

De nombreuses études ont déjà été effectuées pour mettre au point des bactéries lactiques résistantes aux phages.

A cet effet, on peut se référer en particulier aux articles ci-après :
- VLEGELS et al.; Neth. Milk and Dairy J. 43 (1989) 245-259
- SANDERS and KLAENHAMMER. Applied and Environ. Microbiol. (1983) vol. 46, 1125-1133
qui concernent des plasmides qui inhibent l'adsorption des phages ;
- Audrey W. JARVIS ; Applied and Environ. Microbiol. ; March 1988 p. 777-783;
- EP-A₃-0208 468 ;
- COFFEY et al. ; Neth. Milk and Dairy J. 43 (1989) 229-244 ;
- KLAENHAMMER and SANOZKY ; Journal of General Microbiol. (1985), 131, 1531-1541
- DURMAZ et al. J. Bact (1992) 7463-7469
- McLANDSBOROUGH et al. ; Applied and Environ. Microbiol. (1995) 2023-2026
qui décrivent des plasmides qui confèrent une résistance aux phages par le mécanisme d'infection abortive.
- JOSEPHSEN and KLAENHAMMER, Plasmid 23, 71-75 (1990)
- MOINEAU et al. ; Applied and Environ. Microbiol. (1995) 2193-2202
- brevet US 4 883 756
- GAUTIER and CHOPIN ; Applied and Environ. Microbiol. (1987) 53 p. 923-927,
ces derniers articles décrivent notamment des plasmides conférant la résistance aux phages par le mécanisme de restriction/modification.

Dans la demande EP-A1-452 224 une séquence d'ADN comprenant au moins un mécanisme de résistance aux phages est également décrite ; cette séquence d'ADN comporte une partie fonctionnelle du fragment HindIII-HindIII d'environ 3,3 kb du plasmide pPF 144-1 présent dans la souche Escherichia coli déposée le 9 avril 1991 à la C.N.C.M. (Collection Nationale de Cultures de Microorganismes, Institut Pasteur, Paris, France) sous le N°I-1070.

Ce fragment HindIII-HindIII d'environ 3,3 kb, a été isolé à partir du plasmide pPF144 contenu dans la souche Lactococcus lactis ssp lactis, déposée à la C.N.C.M. le 12 avril 1990 sous le N° I-945, laquelle est un transconjugant issu du croisement de la souche donneuse Lactococcus lactis ssp lactis S91 déposée à la C.N.C.M. le 12 avril 1990 sous le N° I-940 et de la souche réceptrice Lactococcus lactis ssp lactis S45, dérivée de la souche Lactococcus lactis ssp lactis C2-LL. décrite par Mc. Kay et al, 1977 dans J. Bacteriol. 257-265. Ce fragment est porteur d'un ou plusieurs mécanismes de résistance aux phages.

A la suite de ces travaux, , une séquence d'ADN de 1,9 kb qui confère à elle seule la résistance aux phages a été isolée à partir de cette séquence d'ADN HindIII-HindIII de 3,3 kb. Cette nouvelle séquence d'ADN d'environ 1,9 kb a été décrite dans EP-A₁-643134.

La demanderesse a maintenant isolé à partir de la souche résistante aux phages S114 de Lactococcus lactis ssp cremoris déposée à la C.N.C.M. le 12 avril 1990 sous le N°I-943, un fragment d'ADN de 7,276 kb à partir du génome de cette souche par digestion partielle à l'aide de l'enzyme de restriction Sau3A. Ce fragment d'ADN à lui seul confère la résistance aux phages. Ce fragment est porteur d'un ou plusieurs mécanismes de résistance aux phages. Ce fragment a été entièrement séquencé. Par la suite, à partir de cette séquence de 7,276 kb la demanderesse a isolé une séquence d'ADN de 817 pb qui confère à elle seule la résistance aux phages.

La présente invention a donc pour objet une nouvelle séquence d'acides nucléiques comprenant au moins un mécanisme de résistance aux phages, ladite séquence ayant 817 pb et étant consitutée par :
a) la séquence d'ADN présentant l'enchainement d'acides nucléiques de SEQ ID No 1 ;
b) les séquences d'ADN hybridant avec la séquence ci-dessus ou un fragment de celle-ci ;
c) les séquences d'ARNm et d'ADNc correspondantes.

La séquence [SEQ ID No 2] est la séquence d'acides aminés déduite de la séquence [SEQ ID N° 1].

La séquence d'ADN [SEQ ID No1] peut être obtenue par la méthode PCR à l'aide des deux oligonucléotides ci-après :

oligonucléotide E [SEQ ID N° 3] :
5' TACGTGAATTCGTAAAAAGTAAAAACGTTAG 3'
EcoRI

oligonucléotide D [SEQ ID N° 4] :
5' TACGTGAATTCTTTTCATAGTCTAGCTATAC 3'
EcoRI

L'invention a particulièrement pour objet la séquence d'ADN comprenant au moins un mécanisme de résistance aux phages qui a l'enchainement d'acides nucléiques [SED ID No 1].

L'invention a également pour objet les séquences d'ADN qui présentent un degré d'homologie élevé avec la séquence d'ADN ci-dessus [SEQ ID N° 1]. Un degré d'homologie élevé signifie ici une homologie (rapport entre les nucléotides identiques et le nombre total de nucléotides) d'au moins 70 %, et de préférence au moins 80 %, des séquences de nucléotides, lorsqu'elles sont alignées d'après l'homologie maximale, selon la méthode d'alignement optimal des séquences de Needleman et Wunsch, 1970, J.Mol. Biol. 48, 443-453. Cette méthode est notamment utilisée dans le logiciel UWGCG de l'Université du Wisconsin : Devereux et al., 1984, Nucl. Ac. Res. 12, 8711-8721 - option GAP.

La présente invention a plus particulièrement pour objet les séquences d'ADN qui s'hybrident avec la séquence d'ADN SEQ ID No.1 ou un fragment de celle-ci. Dans la présente description le terme "hybridation" désigne les conditions classiques d'hybridation et plus particulièrement les conditions d'hybridation strictes.

L'invention a également pour objet les plasmides transformés avec l'une des séquences d'acides nucléiques selon l'invention. Ces plasmides peuvent être par exemple le plasmide pLAB205 dans lequel on a cloné, selon les techniques habituelles bien connues de l'homme du métier, la séquence d'ADN selon l'invention.

L'invention a également trait aux bactéries lactiques résistantes aux phages, de préférence appartenant à l'espèce Lactococcus lactis, qui contiennent au moins une séquence d'acides nucléiques ou un plasmide tel que défini ci-dessus.

Cette séquence d'acides nucléiques ou ce plasmide peuvent avoir été introduits dans les bactéries lactiques par conjugaison, transformation, fusion de protoplastes ou par toute autre méthode de transfert de gènes bien connue de l'homme du métier.

Les bactéries lactiques qui peuvent avantageusement être transformées à l'aide de la séquence d'acides nucléiques selon l'invention ou d'un plasmide la contenant sont par exemple les souches Lactococcus lactis ssp cremoris, Lactococcus lactis ssp lactis, Lactococcus lactis ssp lactis var. diacetylactis.

Ces souches ainsi transformées peuvent être utilisées pour transmettre par conjugaison, transformation, transduction, fusion de protoplastcs ou toute autre méthode de transfert de gènes bien connue de l'homme du métier, un mécanisme de résistance aux phages à une souche d'intérêt industriel. Ce mécanisme peut être porté par un plasmide ou par une autre partie du génome de la bactérie. Lorsque celui-là est porté par un plasmide il est avantageux de le transférer par conjugaison.

L'invention a également trait aux souches d'intérêt industriel résistantes aux phages ainsi obtenues.

L'invention sera mieux comprise à l'aide des exemples ci-après, qui comprennent des résultats expérimentaux et une discussion de ceux-ci. Certains de ces exemples concernent des expériences effectuées dans le but de réaliser l'invention, d'autres des exemples de réalisation de l'invention donnés bien sûr à titre purement illustratif.

Une grande partie de l'ensemble des techniques décrites dans ces exemples, bien connues de l'homme de l'art, est exposée en détail dans l'ouvrage de Sambrook Fritsch et Maniatis : "Molecular cloning ; a Laboratory Manual" publié en 1989 par les éditions Cold Spring Harbor Press à New York (2e édition).

### Exemple 1 : Obtention du fragment de 7,276 kb

La souche S114 de Lactococcus lactis ssp cremoris déposée à la C.N.C.M. sous le N° I-943 le 12 Avril 1990, contient une ou plusieurs résistances aux phages. En particulier, elle a transféré par conjugaison dans la souche réceptrice Lactococcus lactis S45, dérivée de la souche Lactococcus lactis C2-LL décrite par McKay et al., 1977, J. Bacteriol. 257-265, le plasmide pPF66 qui confère la résistance aux phages ⌀ 53 (groupe I) et ⌀ 59 (groupe III). D'autres mécanismes de résistance aux phages peuvent être présents dans la souche I-943. Cest pourquoi une banque d'ADN a été construite à partir du génome de la souche I-943.

L'ADN de la souche I-943 a été extrait et digéré partiellement avec l'enzyme de restriction Sau3A. Le plasmide pLDP1 a été utilisé. Le plasmide pLDP1 dérive du plasmide pVA838 (Macrina FL. et al, gene 19, 345-353) par délétion du fragment de 1523 pb compris entre le site HindIII (0) et le site EcoRI (1523) et remplacement de celui-ci par 54 paires de bases correspondant aux sites multiples de clonage du plasmide pUC18 (Yanisch-Perron C. et al : 1985 - gene 33, 103-119) bordés par EcoRI-HindIII. Le plasmide pLDP1 est digéré au site BamHI et les extrémités cohésives sont déphosphorylées grâce à la phosphatase alcaline. Le mélange plasmide et fragments Sau3A est ligaturé avec la DNA ligase T4 et sert à transformer la souche TG1 de E.coli (sélection sur milieu LB + érythromycine à 200 µg/ml). 100.000 clones sont obtenus.

Les 100.000 clones ont été mélangés, l'ADN plasmidique total a été extrait et a servi à transformer la souche Lactococcus lactis MG 1363 décrite par GASSON M.J. (1983) dans J. Bacteriol.154 : 1-9, dénommée ci-après souche L.lactis S56 ou S56. Les transformants ont été étalés sur des boîtes de M17 + érythromycine 5 µg/ml + glucose 0,5 % à raison de 500 clones par boîte et laissés à l'étuve à 30° C pendant 30 h. Le milieu M17 est décrit par TERZAGHI et al (1975), dans Appl. Environ Microbiol. 29, 807-813. Puis ces clones ont été répliqués par la technique de réplique sur velours sur des boîtes de M17 + glucose 0,5 % sur lesquelles 10⁸ phages ⌀ 59 (groupe III) ont été étalés par boîte. Par ce procédé, on a trouvé 6 clones résistants au phage ⌀ 59 sur 3600 clones testés. L'un de ces clones a été testé par lysotypie et montre une résistance totale à ⌀ 59 et une résistance partielle à ⌀ 53. Le plasmide pLDP1 présent dans ce clone contient un fragment d'environ 7,5 kb. Ce nouveau plasmide comprenant pLDP1 et ce fragment d'environ 7,5 kb a été appelé pLAB201. La séquence d'acides nucléiques de ce fragment a été déterminée par la méthode de Sanger et al. (PNAS - USA, 14, 5463-1977) : sa taille est de 7,276 kb.

L'analyse de la séquence a montré que ce fragment de 7,276 kb possède 10 phases de lecture ouverte (ORF) d'une taille supérieure à 300 paires de base.

### Exemple 2 : Traitement à l'exonucléase BAL31

Un site unique de digestion SalI est présent dans pLDP1, à côté de l'ORF1. Le plasmide pLAB201 a été digéré par SalI et a été traité à l'exonucléase BAL31. L'ADN traité à BAL31 a été religaturé par la DNA ligase T4 et a retransformé TG1 (sélection érythromycine 200 µg/ml). L'ensemble des plasmides obtenus a été extrait et a servi à transformer S56 (sélection érythromycine 5 µg/ml). Des comparaisons ont été faites entre la taille et la localisation de la délétion à BAL31 et la perte ou le maintien du phénotype de résistance. On constate que la résistance aux phages disparaît lorsque la phase de lecture ouverte, appelée ORF7 est délétée.

### Exemple 3 : Amplification par PCR d'un fragment interne du fragment de 7,276 kb

La technique de la PCR (Polymerase Chain Reaction), décrite par exemple dans l'ouvrage de Maniatis, déjà cité, permet d'amplifier un fragment d'ADN compris entre deux oligonucléotides convenablement choisis. Cet ADN amplifié peut être aisément cloné si des sites de restriction sont apportés par les oligonucléotides. En effet, les séquences de ces oligonucléotides peuvent comporter, à leur extrémité 5', une partie hétérologue de l'ADN à amplifier, constituée par exemple de 10 à 12 paires de bases, dont 6 constituent un site de restriction.

Dans cet exemple, les oligonucléotides suivants ont été synthétisés : 
Oligonucléotide D [SEQ N° 4]
5' TACGTGAATTCTTTTCATAGTCTAGCTATAC 3'
EcoRI
 
Oligonucléotide E [SEQ N° 3]
5' TACGTGAATTCGTAAAAAGTAAAAACGTTAG 3'
EcoRI
 Les oligonucléotides E et D ont permis d'amplifier un fragment d'ADN de 817 pb comportant l'ORF7.

Cet ADN a été amplifié sous la forme d'un fragment EcoRI-EcoRI grâce aux sites de restriction apportés par les oligonucléotides, permettant un clonage dans le plasmide navette pLDP1.

Ce fragment d'ADN a été amplifié par PCR à partir de l'ADN total de la souche I-943. Les produits de PCR ont été purifiés par une extraction au phénol-chloroforme, digérés par EcoRI et clonés dans le vecteur pLDP1.

Le clonage des fragments dans pLDP1 a permis de les introduire, après amplification des plasmides recombinants dans la souche TG1 d'E.coli, dans une souche de L.lactis S56 et de déterminer s'ils confèrent la résistance aux phages.

### Exemple 4 : Résistance aux phages conférée par le fragment de 817 pb

Les plasmides pLAB205 et pLDP1 ont été introduits dans la souche S56 de L.lactis. La résistance aux phages des clones obtenus a été testée en réalisant une titration (UFP/ml) avec les phages ⌀ 53 et ⌀ 59.

Les résultats sont donnés ci-après :

| Souche | Phage ⌀ 53 (I) | | Phage ⌀ 59 (III) | |
|---|---|---|---|---|
| | Titre | Taille des plages (mm) | Titre | Taille des plages (mm) |
| S56 | 4.10⁹ | 3 | 6.10⁸ | 2 |
| S56 (pLDP1) | 4.10⁹ | 3 | 4.10⁸ | 2 |
| S56 (pLAB205) | 2.10⁸ | 0,5 | 6.10³ | 0,5 |
| UFP/ml = unités formant plage par ml | | | | |

## Revendications

1. Séquence d'acides nucléiques comprenant au moins un mécanisme de résistance aux phages, caractérisée en ce qu'elle est constituée par :
a) la séquence d'ADN présentant l'enchainement d'acides nucléique s[SEQ ID No 1];
b) les séquences d'ADN hybridant avec la séquence ci-dessus ou un fragment de celle-ci ;
c) les séquences d'ARNm et d'ADNc correspondantes.

2. Séquence d'ADN comprenant au moins un mécanisme de résistance aux phages, caractérisée en ce qu'elle a la séquence [SEQ ID N° 1] ou une séquence présentant un degré d'homologie élevé avec ladite séquence [SEQ ID N° 1].

3. Plasmide comprenant au moins un mécanisme de résistance aux phages, caractérisé en ce qu'il comporte une séquence d'acides nucléiques selon la revendication 1.

4. Plasmide comprenant au moins un mécanisme de résistance aux phages, caractérisé en ce qu'il comporte une séquence d'ADN selon la revendication 2.

5. Bactérie lactique résistante aux phages, caractérisée en ce qu'elle contient au moins un plasmide selon l'une des revendications 3 ou 4.

6. Utilisation des bactéries lactiques selon la revendication 5, pour conférer un mécanisme de résistance aux phages à des souches d'intérêt industriel.
